# EUROPEAN PATENT SPECIFICATION

(11) **EP 4 272 795 B1**
(45) Date of publication and mention of the grant of the patent: **24.12.2025**
(21) Application number: 21913562.1
(22) Date of filing: 17.11.2021
(51) Int. Cl.: A61M 25/01, A61B 1/00, A61B 1/005

(54) **ADJUSTABLE BENT CATHETER**
EINSTELLBARER GEBOGENER KATHETER
CATHÉTER COURBÉ RÉGLABLE

(30) Priority: 31.12.2020 CN 202011619716
(43) Date of publication of application: 08.11.2023
(73) Proprietor: Lifetech Scientific (Shenzhen) Co., Ltd., Shenzhen, Guangdong 518000 (CN)
(72) Inventor: GUAN, Chengmei, Guangdong 518000 (CN); LIU, Xiaoying, Guangdong 518000 (CN); WANG, Gang, Guangdong 518000 (CN)
(74) Representative: Michalski Hüttermann & Partner Patentanwälte mbB
(86) International application number: PCT/CN2021/131171
(87) International publication number: WO 2022/142819

(56) References cited:
- WO-A1-2020/123774
- CN-A- 101 708 130
- CN-A- 104 782 331
- CN-A- 105 960 311
- CN-A- 111 246 908
- CN-A- 111 246 908
- CN-U- 206 494 647
- CN-U- 211 314 043
- CN-U- 211 935 127
- US-A1- 2014 005 601
- US-B1- 6 440 062

## Description

### Technical Field

The present application belongs to the technical field of medical devices, and particularly relates to a deflectable catheter.

### Background Art

Endovascular techniques are emerging medical procedures that rely on a catheter to be moved along the lumen of a vessel to the lesion site (e.g., coronary artery, carotid artery, celiac trunk artery, renal artery, etc.) and then to conduct minimally invasive treatment at the lesion in vivo by the implantation of a stent or drug or other implanted devices, where the catheter is a tool to create an access from extracorporeal to intracorporeal in endoluminal procedures. Because of the variability and complexity of the vasculature of peripheral vessels such as iliac artery, femoral artery, common carotid artery, innominate trunk and coronary artery, as well as aortic vessels, there are some difficulties in positioning the implanted devices accurately.

At present, most of the catheters on the market have their tips pre-shaped into different curved shapes, such as flexor sheath, shuttle sheath, and bent sheath, depending on the vascular path characteristics at the target lesion site. However, when treating multiple vascular diseases or diseases with complex vascular anatomy, it is necessary to prepare a large number of catheters, which increases the consumption of devices and operation time.

In order to solve the above problems, deflectable catheters are put into market. A deflectable catheter has a deflectable distal part, thus increasing its applicability and avoiding spending a lot of time selecting the catheter model during the surgery.

At present, the deflectable catheters on the market are mostly unidirectionally deflectable catheters, of which the sheath tips can only deflect in a single direction. For the target sites with vascular distortion and varied vascular opening positions, the unidirectional deflection usually fails to reach the expected position and angle in a single step. The positioning of the catheters can be difficult, and it is necessary to adjust the sheaths by external rotation. Sometimes it is even necessary to substitute a different catheter and make multiple adjustments, which increases the complexity of surgical operation and possibly leads to complications.

D1(US 2014/005601 A1) provides a bi-directional catheter with nearly double the throw in its catheter tip deflection. In particular, the travel path of each the puller wire includes a U-turn or doubling-back around a pulley which minimizes the offset angle between the puller wire and the longitudinal axis of the control handle while maximizing the travel distance of that puller wire for any given distance traveled by the pulley drawing the puller wire. In one embodiment, the catheter has an elongated catheter body, a catheter tip section with first and second diametrically-opposed off-axis lumens, and a control handle which includes a steering assembly having a lever structure carrying a pair of pulleys for simultaneously drawing and releasing corresponding puller wires to deflect the tip section of the catheter.

D2 (CN 111246908 A1) provides a steerable medical apparatus includes a shaft (14), a steering mechanism (38), and an actuation mechanism (48). The shaft has a proximal portion (16), a distal portion (18), a first pull wire (20), and a second pull wire (22). The distal ends of the first (20b) and second (22b) pull wires are coupled to the distal portion of the shaft. The steering mechanism has a first wheel (40) and a second wheel (42) coupled by a differential mechanism (44). The proximal ends of the first (20a) and second (22a) pull wires are respectively coupled to the first and second wheels. The actuation mechanism iscoupled to the steering mechanism. Actuating the actuation mechanism in a first operational mode causes the distal portion of the shaft to curve in a first plane. Actuating the actuation mechanism ina second operational mode causes the distal portion of the shaft to curve away from the first plane.

D3 (WO 2020/123774 A1) provides a steerable sheath with a deflection mechanism assembly is provided. The assembly includes a tubular shaft that receives first and second longitudinal movement wires at a distal end. A control handle includes a main body configured to receive first and second bevel gears. The first longitudinal movement wire is coupled to the first bevel gear and the second longitudinal movement wire is coupled to the second bevel gear. A rotatable adjustment knob is engageable with the control handle and has an external geared portion matingly engageable with the first and second bevel gears and the rotatable adjustment knob is moveable between a first and second position.

### Summary of the Invention

An object of the present application is to provide a deflectable catheter which is intended to solve the problem that the deflectable catheters in the prior art can only realize unidirectional deflection but cannot realize bidirectional deflection.

The present application is implemented as follows: a deflectable catheter including:
a sheath having a bendable section;
a first traction wire anchored to the bendable section of the sheath;
a second traction wire anchored to the bendable section of the sheath at a location circumferentially different from that of the first traction wire; and
a driving mechanism connected to both the first traction wire and the second traction wire, where the driving mechanism can pull and release the first traction wire and the second traction wire, and when the driving mechanism pulls any of the first traction wire and the second traction wire, the driving mechanism will synchronously release the other one of the first traction wire and the second traction wire,
an adjusting member (40) and a handle housing (50);
wherein the adjusting member is configured to adjust the driving mechanism (30) for selecting and adjusting a bending direction and a bending angle of the sheath (10), the adjusting member (40) comprises an adjusting component (41) which is at least partially exposed outside the handle housing (50), and
the deflectable catheter (100) further comprises a stop mechanism (70) arranged opposite to the adjusting component (41) and is configured to maintain the bendable section (11) of the sheath (10) in a bent state.

A deflectable catheter provided in an embodiment of the present application is provided with a sheath having a bendable section, a first traction wire and a second traction wire anchored to the bendable section of the sheath, and a driving mechanism configured to pull and release the first traction wire and the second traction wire, wherein when the driving mechanism pulls any of the first traction wire and the second traction wire to bend the sheath towards the side of the pulled traction wire, the other traction wire is correspondingly released, so that the sheath is bidirectionally bendable at its bendable section in at least two directions of the sheath. In this way, the requirements for interventional treatment of complex lesion sites, for example, with vascular distortion and varied vascular opening positions, may be fully met, and the frequency of catheter adjustments and replacements by clinical operators may be greatly reduced, thus facilitating the operation and reducing the risk of complications.

### Brief Description of the Drawings

Fig. 1 is a schematic front view of a deflectable catheter according to an embodiment of the present application;
Fig. 2 is a schematic view of a deflectable catheter with a part of handle housing removed according to an embodiment of the present application;
Fig. 3 is a schematic view of a handle housing with a part of housing removed according to an embodiment of the present application;
Fig. 4 is a perspective view of an anchoring ring according to an embodiment of the present application;
Fig. 5 is a schematic view of an anchoring ring being connected to a first traction wire and a second traction wire according to an embodiment of the present application;
Fig. 6 is a schematic view of a sheath of a deflectable catheter according to a further embodiment of the present application;
Fig. 7 is a schematic view of an assembly of a driving mechanism and an adjusting member of a deflectable catheter according to the first embodiment of the present application;
Fig. 8 is an exploded view of a driving mechanism and an adjusting member of a deflectable catheter according to the first embodiment of the present application;
Fig. 9 is a perspective view of a support frame of a driving mechanism according to the first embodiment of the present application;
Fig. 10 is a perspective view of a first driven bevel gear of a driving mechanism according to the first embodiment of the present application;
Fig. 11 is a schematic view showing an assembly of a first driven bevel gear and a hood of a driving mechanism according to the first embodiment of the present application;
Fig. 12 is a perspective view of a hood of a driving mechanism according to the first embodiment of the present application;
Fig. 13 is an exploded view of an adjusting member according to the first embodiment of the present application;
Fig. 14 is a schematic view showing a handle housing of a deflectable catheter with the bottom housing and the upper housing exploded according to the first embodiment of the present application;
Fig. 15 is a schematic view of an assembly of a driving mechanism, an adjusting member, and a stop mechanism according to the first embodiment of the present application;
Fig. 16 is an exploded view of a stop mechanism according to the first embodiment of the present application;
Fig. 17 is a perspective view of a stop plate of a stop mechanism according to the first embodiment of the present application;
Fig. 18 is a cross-sectional view of a stop pin of a stop mechanism according to the first embodiment of the present application;
Fig. 19 is an exploded view of a connecting sleeve, a T-joint, a guide sleeve, and a tail sleeve according to the first embodiment of the present application; and
Fig. 20 is a perspective view of a T-joint according to the first embodiment of the present application.

### Detailed Description of the Invention

In order to make the objects, technical schemes, and advantages of the present application clearer, the present application will be further illustrated in detail below with reference to the drawings and embodiments. It is understood that the particular embodiments described herein are illustrative only and are not intended to limit the present application.

It is noted that when an element is referred to as being "fixed" or "arranged" on another element, it can be directly on the other element or an intervening element(s) may be present as well. When an element is referred to as being "connected" to another element, it can be directly connected to the other element or an intervening element(s) may be present as well.

It is further noted that the directional terms left, right, upper, lower, and the like in the embodiment are merely relative concepts to each other or references are made to the normal use of the product, and therefore theses terms should not be considered as limiting.

In order to describe the structures of the embodiments of the present application more clearly, the terms "proximal end" and "distal end" are defined herein as customary terms used in the field of interventional medicine. Specifically, a "distal end" refers to an end that is distal to the operator during the surgical operation and a "proximal end" refers to an end that is proximal to the operator during the surgical operation.

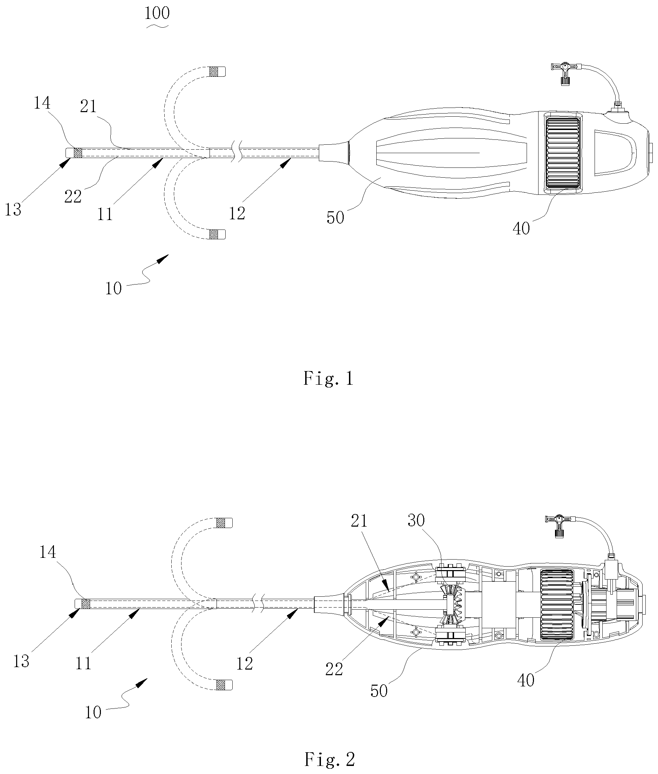

As shown in Figs. 1-21, various related drawings of the deflectable catheter are provided in the embodiments of the present application.

Referring to Figs. 1 to 3, the deflectable catheter 100 provided in an embodiment of the present application includes a sheath 10 having a bendable section 11, a first traction wire 21 and a second traction wire 22 anchored to the bendable section 11 of the sheath 10, a driving mechanism 30 connected to both the first traction wire 21 and the second traction wire 22, an adjusting member 40, and a handle housing 50, where the driving mechanism 30 can pull and release the first traction wire 21 and the second traction wire 22, and when the driving mechanism 30 pulls any of the first traction wire 21 and the second traction wire 22, the sheath 10 bends towards the side of the pulled traction wire, and at the same time, the driving mechanism 30 will synchronously release the other traction wire which is not pulled correspondingly with the bending of the sheath 10. For example, as shown in Fig. 2, when the driving mechanism 30 pulls the first traction wire 21 to bend the distal end of the sheath 10 towards the side of the first traction wire 21, the second traction wire 22 is released in length with the bending of the sheath 10; when the driving mechanism 30 pulls the second traction wire 22 and drives the sheath 10 to bend towards the side of the second traction wire 22, the first traction wire 21 is released in length with the bending of the sheath 10. In this way, the cooperation of the driving mechanism 30, the first traction wire 21, and the second traction wire 22 enables the sheath 10 to bend in two directions at the bendable section 11, thus achieving bidirectional bending.

The deflectable catheter 100 provided in the present application is provided with a sheath 10 having a bendable section 11, a first traction wire 21 and a second traction wire 22 anchored to the bendable section 11 of the sheath 10, and a driving mechanism 30 configured to pull and release the first traction wire 21 and the second traction wire 22, wherein when the driving mechanism 30 pulls any of the first traction wire 21 and the second traction wire 22, the sheath 10 bends towards the side of the pulled traction wire, and at the same time, the other traction wire is released synchronously, so that the sheath 10 is bidirectionally bendable at its bendable section 11 in at least two directions of the sheath 10, so as to fully meet the requirements for interventional treatment of complex lesion sites, for example, with vascular distortion and varied vascular opening positions, so that the frequency of catheter adjustments and replacements by clinical operators can be greatly reduced, thus facilitating the operation and reducing the risk of complications.

As a specific embodiment, the sheath 10 further includes a first hard section 12 and a second hard section 13, and the bendable section 11 is located between the first hard section 12 and the second hard section 13. The first hard section 12 is located at the proximal end of the sheath 10, the second hard section 13 is located at the distal end of the sheath 10, and the bendable section 11 is located near the distal end of the sheath 10. In this way, it can be ensured that the sheath 10 has some stiffness during in vivo delivery to reduce the contact with human tissues to avoid scratching, and that the sheath 10 can bend at a corresponding position where a bending delivery channel is desired at the tissue with a lesion. The bendable section 11 may be made in a structure having a metal stent as a stent body and having biological materials, such as a Teflon film, coated on the surface.

As a specific embodiment, a wire passing channel (not shown) for receiving the first traction wire 21 and the second traction wire 22 is provided in the side wall of the sheath 10, so as to provide a special moving channel for the first traction wire 21 and the second traction wire 22, thereby ensuring their smooth movement, and also providing protection for them from interference from the outside.

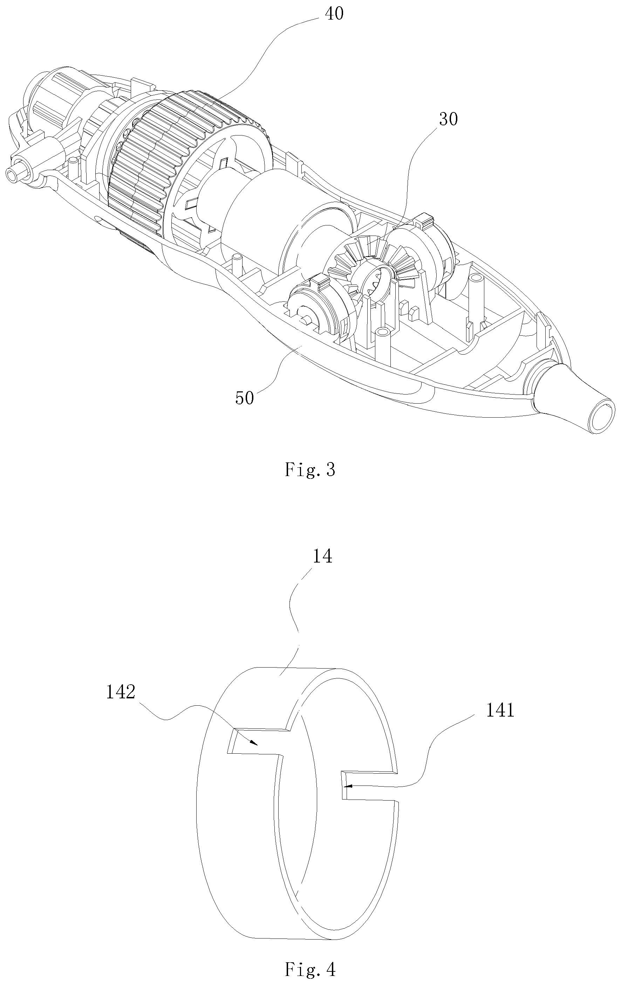

In the embodiment of Fig. 4, the first traction wire 21 and the second traction wire 22 are fixedly connected to the bendable section 11 of the sheath 10 via an anchoring ring 14, and the anchoring points of the first traction wire 21 and the second traction wire 22 with the anchoring ring 14 are spaced apart, so that the first traction wire 21 and the second traction wire 22 can guide the sheath 10 to bend in two different directions on different radial planes of the same circumference of the bendable section 11 of the sheath 10.

Of course, as an alternative embodiment, the first traction wire 21 and the second traction wire 22 may be located in the same radial plane of the anchoring ring 14, i.e., in the same radial plane of the same circumference of the bendable section 11 of the sheath 10 (not shown). In this way, the first traction wire 21 and the second traction wire 22 can bend in two completely opposite directions on the same circumference of the bendable section 11 of the sheath 10, so that a suitable catheter can be selected according to individual differences in physiological and anatomical structures of different patients, and the bending curvature of the bendable section 11 of the sheath 10 can be adjusted, such as small curve, medium curve, and large curve, so as to meet the requirements of different human bodies, thereby reducing the number of punctures and injuries to the human body, simplifying the surgical process, and shortening the operation time.

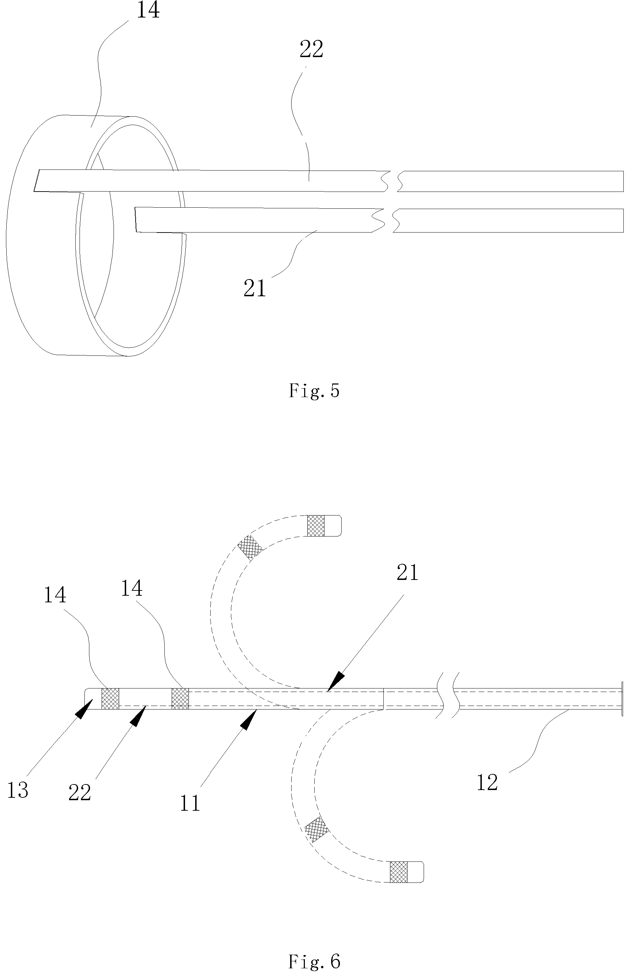

In the embodiment of Figs. 4 and 5, notches 141 and 142 are symmetrically arranged on the side wall of the anchoring ring 14, and the distal ends of the first traction wire 21 and the second traction wire 22 are fixedly connected in the pair of notches 141 and 142, separately, which can be specifically completed by welding or affixing. The first traction wire 21 and the second traction wire 22 can be arranged in a flat shape or a round shape. When arranged in the flat shape, the outer surfaces and the inner surfaces of the flat traction wires do not protrude from the inner and outer side walls of the anchoring ring 14, so that the stress impact to which the first traction wire 21 and the second traction wire 22 are subjected may be reduced, thereby ensuring the stability of the connection with the anchoring ring 14. The embodiment may avoid the traction wires protruding from the surface of the anchoring ring, so as to avoid the risk of wire leakage or ring leakage during the processing and hot melting of the sheath. In the related art, there is some connection where, after the traction wire is folded in half, the anchoring ring runs through the traction wire, so as to realize the connection between the anchoring ring and the traction wire. Such connection would cause the anchoring ring to be subjected to the shear stress of the traction wire during the deflection process, which would bring the risk of anchoring ring breakage or deflection wire breakage due to the concentration of the shear stress. In the deflectable catheter provided in the embodiment of the present application, by providing notches 141 and 142 on the anchoring ring 14 and then fixing the traction wires at the notches 141 and 142 of the anchoring ring 14 by means of welding, and the like, the anchoring ring 14 and the traction wires are fixedly connected, which not only avoids the risk of wire leakage or ring leakage, but also provides a more reliable welding and higher security.

As a further alternative embodiment, referring to Fig. 6, it is also possible to provide two anchoring rings 14 spaced apart on the bendable section 11 of the sheath 10, to which the first traction wire 21 and the second traction wire 22 are anchored, separately. In this way, the first traction wire 21 and the second traction wire 22 can pull the sheath 10 to bend in two different directions in radial directions of different circumferential directions of the bendable section 11 of the sheath 10, which may also meet clinical needs. Such a structure is particularly suitable for use in cases where there are bifurcated branches in different sections of the main vascular channel.

The driving mechanism 30 includes a driving member (the driving member is a driving bevel gear 31 in the embodiment), a first driven member linked with the driving member (the first driven member is a first driven bevel gear 32 in the embodiment), and a second driven member linked with the driving member (the second driven member is a second driven bevel gear 33 in the embodiment), where the first driven member is connected to the first traction wire, the second driven member is connected to the second traction wire, and the driving member drives any of the first driven member and the second driven member to pull the traction wire connected thereto and synchronously drives the other one of the first driven member and the second driven member to release the traction wire connected thereto, thereby enabling bidirectional deflection. It is noted that in other embodiments, the driving member may be a worm, the first driven member may be a worm gear that engages the worm, and the second driven member may be another worm gear that engages the worm.

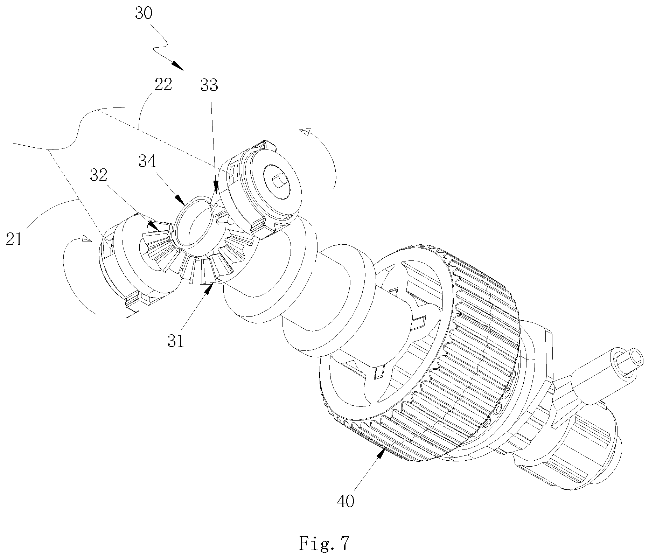

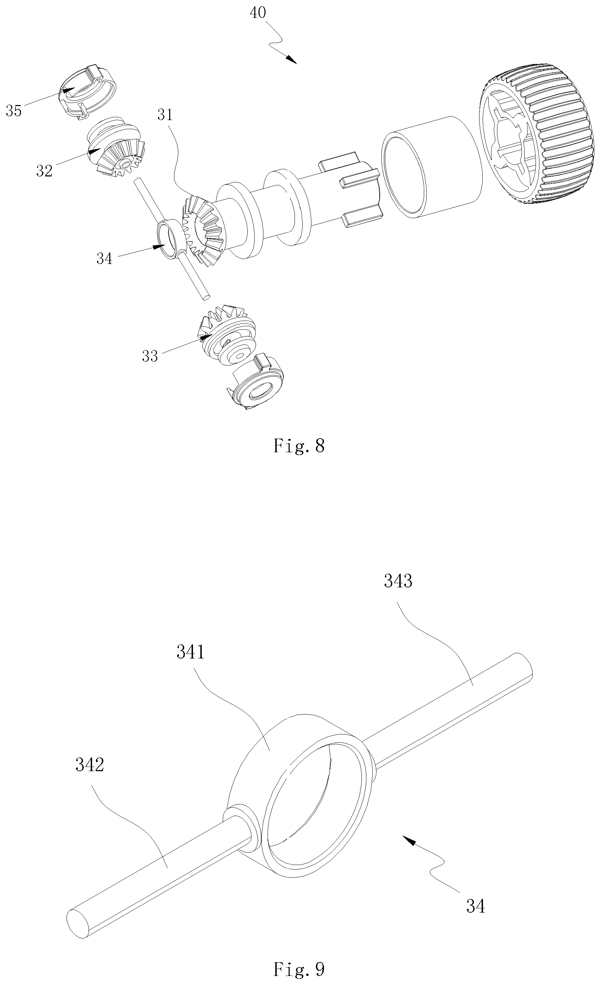

As a first embodiment, referring to Figs. 7 and 8 in combination, the driving bevel gear 31 and the sheath 10 are arranged coaxially, the first driven bevel gear 32 and the second driven bevel gear 33 mesh with the driving bevel gear 31, the first driven bevel gear 32 is connected to the first traction wire 21, and the second driven bevel gear 33 is connected to the second traction wire 22. In the embodiment of the present application, the first driven bevel gear 32 and the second driven bevel gear 33 are arranged symmetrically with respect to at least one axial plane of the driving bevel gear 31, i.e., the first driven bevel gear 32 and the second driven bevel gear 33 are located at circumferentially different positions of the driving bevel gear 31. In this way, the driving bevel gear 31, the first driven bevel gear 32, and the second driven bevel gear 33 have a structural stability and a relatively stable transmission. Meanwhile, the first driven bevel gear 32 and the second driven bevel gear 33 can be ensured to synchronously drive the first traction wire 21 and the second traction wire 22 to be received or released, respectively. When the driving bevel gear 31 drives any of the first driven bevel gear 32 and the second driven bevel gear 33 to rotate to further pull the traction wire connected thereto correspondingly, the other one of the first driven bevel gear 32 and the second driven bevel gear 33 can be synchronously driven to rotate to correspondingly release the traction wire connected to the other driven bevel gear. In this way, the driving bevel gear 31, the first driven bevel gear 32, the second driven bevel gear 33, the first traction wire 21, and the second traction wire 22 can cooperate and coordinate with each other, such that the sheath 10 is able to bidirectionally bend, and the first traction wire 21 and the second traction wire 22 can be synchronously received and released.

As a specific embodiment, the rotational central axis of the first driven bevel gear 32 is arranged coaxially with that of the second driven bevel gear 33, and the rotational central axes of the first driven bevel gear 32 and the second driven bevel gear 33 are both perpendicular to the central axis of the driving bevel gear 31. In this way, the radial stress applied to the driving bevel gear 31 by the first driven bevel gear 32 and the second driven bevel gear 33 in the radial direction will be more balanced such that the structure of the whole bevel gear transmission system will be more stable and the transmission of torque will be more balanced, so that the synchronism of pulling and releasing the first traction wire 21 and the second traction wire 22 will be better.

In the embodiment, since the first driven bevel gear 32 and the second driven bevel gear 33 both mesh with the driving bevel gear 31, when the driving bevel gear 31 drives the first driven bevel gear 32 and the second driven bevel gear 33 to rotate simultaneously, the rotation directions of the first driven bevel gear 32 and the second driven bevel gear 33 will be opposite, and at this point, the first traction wire 21 is wound on the first driven bevel gear 32 in a counterclockwise direction, and the second traction wire 22 is also wound on the second driven bevel gear 33 in a counterclockwise direction, i.e., the winding directions of the first traction wire 21 and the second traction wire 22 are the same (i.e., both are wound in a clockwise direction, or both are wound in a counterclockwise direction), so that with the driving force of the driving bevel gear 31, when the first traction wire 21 is pulled, the second traction wire 22 is released, and vice versa. At this point, the synchronous control of the first traction wire 21 and the second traction wire 22 is enabled only with one bevel gear and two driven bevel gears, which facilitates a compact structure which occupies a small space occupation, thereby effectively meeting the requirements that the medical devices should not only achieve accurate control, but also avoid being too large in size to manipulate. Of course, in other embodiments, the rotation directions of the first driven bevel gear 32 and the second driven bevel gear 33 can be the same, and the winding directions of the first traction wire 21 and the second traction wire 22 can be opposite. For example, one of the first traction wire 21 and the second traction wire 22 is wound in a clockwise direction, and the other of the first traction wire 21 and the second traction wire 22 is wound in a counterclockwise direction.

In the embodiment of Figs. 8 and 9, the driving mechanism 30 further includes a support frame 34. As a specific embodiment, the support frame 34 includes a support ring 341, a first rotary shaft 342, and a second rotary shaft 343, where the first rotary shaft 342 and the second rotary shaft 343 are both fixedly connected to the support ring 341. The first rotary shaft 342 and the second rotary shaft 343 are coaxially arranged, and the first rotary shaft 342 and the second rotary shaft 343 are located in the same radial plane of the sheath 10, where the first rotary shaft 342 is rotatably connected to the central part of the first driven bevel gear 32, the second rotary shaft 343 is rotatably connected to the central part of the second driven bevel gear 33, and the central axis of the driving bevel gear 31 passes through the center of the support ring 341 of the support frame 34. In this way, it is structurally ensured that the central axes of the first driven bevel gear 32 and the second driven bevel gear 33 are always on the same central axis, so that the first traction wire 21 and the second traction wire 22 are kept moving as synchronously as possible. As an embodiment, the support frame 34 may be made of a metallic material with good rigidity to better withstand the great radial stresses of the first driven bevel gear 32 and the second driven bevel gear 33. In a specific implementation, the rotatably contacting surfaces of the first rotary shaft 342 with the first driven bevel gear 32 are relatively smooth surfaces, and the rotatably contacting surfaces of the second rotary shaft 343 with the second driven bevel gear 33 are also relatively smooth surfaces. Here, a "smooth surface" means that the roughness does not exceed 6.4 Ra. The rotatably contacting surfaces of the first rotary shaft 342 with the first driven bevel gear 32 and the rotatably contacting surfaces of the second rotary shaft 343 with the second driven bevel gear 33 have low degree of roughness to effectively reduce the friction between the contacting surfaces, so that both the first rotary shaft 342 and the second rotary shaft 343 can rotate relatively smoothly, thereby reducing the driving force needed for driving their rotation, so as to achieve the purpose of saving time and effort, and also improving the reliability of the entire driving mechanism 30.

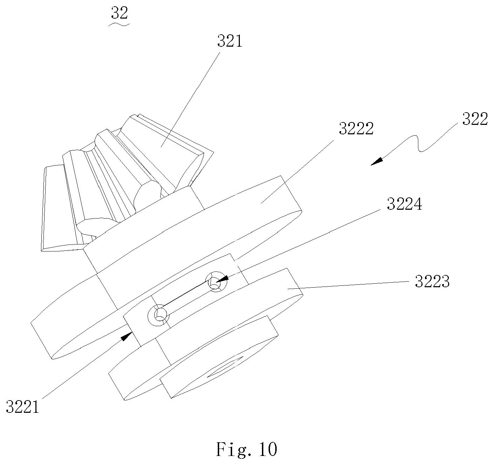

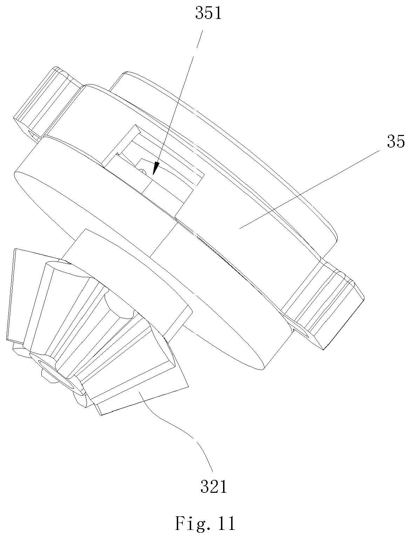

In the embodiment of Figs. 10 and 11, the first driven bevel gear 32 includes a gear portion 321 meshing with the driving bevel gear 31 and a wire spool 322 connected to the gear portion 321, and the proximal end of the first traction wire 21 is fixedly connected to the wire spool 322. Specifically, the wire spool 322 includes a cylindrical wire reel 3221 and a first fence 3222 and a second fence 3223 convexly provided on the outer surface of the wire reel 3221, where the first fence 3222 and the second fence 3223 are spaced apart. The first traction wire 21 can be wound around a part of the wire reel 3221 between the first fence 3222 and the second fence 3223, and a wire passing hole 3224 is provided on the wire spool 322 to allow the traction wire to run through. The rotation of the first driven bevel gear 32 will drive the first traction wire 21 that is fixedly connected to the wire spool 322 to be taken back or released around the wire spool 322, thereby enabling the receipt and release of the first traction wire 21.

As a specific embodiment, the specific structure of the second driven bevel gear 33 and the connection relationship between the second traction wire 22 and the second driven bevel gear 33 can be identically designed with reference to the structure of the first driven bevel gear 32, so it will not be described in detail herein.

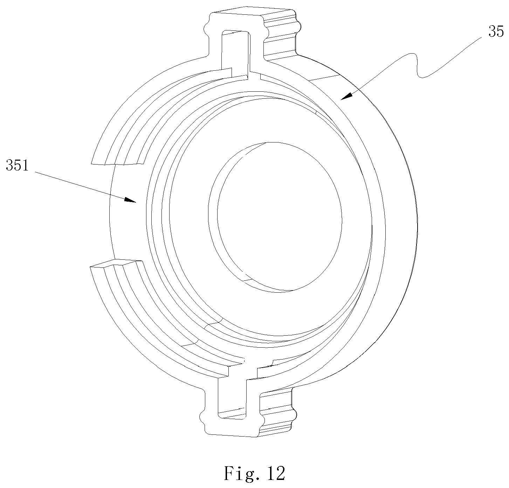

As a specific embodiment, referring also to Fig. 12, the wire spool 322 is surrounded by a cover 35 for limiting the first traction wire 21, and the cover 35 is provided with an opening 351 for the first traction wire 21 to extend therethrough. More specifically, the cover 35 surrounds the wire reel 3221 of the wire spool 322 and encloses the wire spool 322 to form a separate space. The cover 35 may limit the movement of the first traction wire 21 in the separate space enclosed by the wire spool 322 and the cover 35, thereby preventing the first traction wire 21 from jumping off and being mistakenly wound around other components, which may cause a wire jam.

As an embodiment, a corresponding cover may be provided on the second driven bevel gear 33 as well to prevent jamming of the second traction wire 22, which will not be described in detail herein.

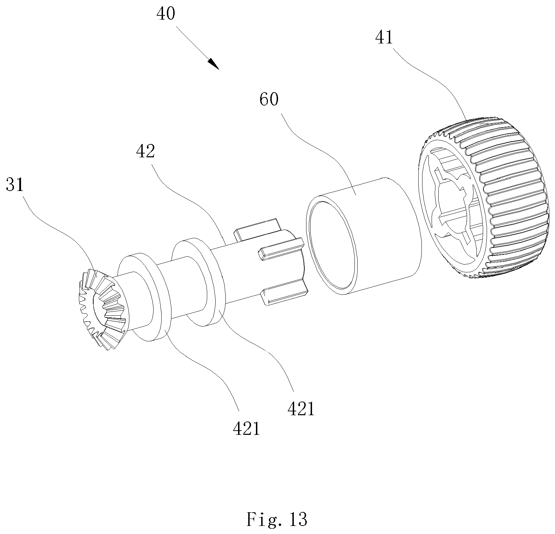

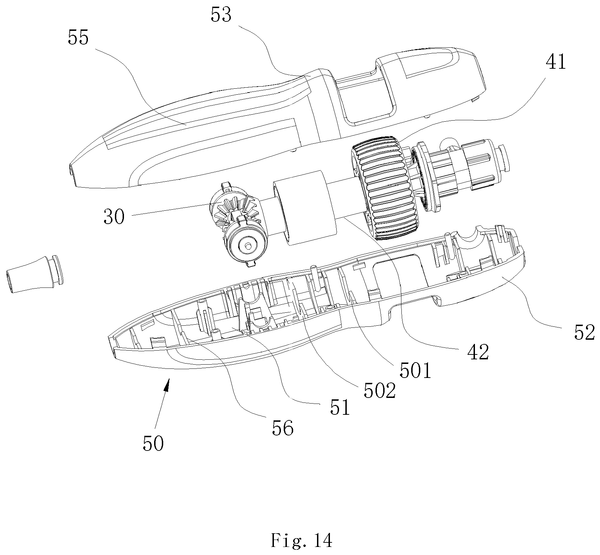

Referring to Figs. 13 and 14, the embodiment of the present application provides an adjusting member 40 for adjusting the driving mechanism 30 so as to select and adjust the bending direction and bending angle of the sheath 10. The adjusting member 40 includes an adjusting component 41 which is at least partially exposed outside the handle housing 50. The adjusting component 41 may be specifically a knob, and the adjusting component 41 may be partially or entirely exposed outside the handle housing 50. In this way, for the complex lesion sites with vascular distortion and varied vascular openings, the bending direction and bending angle of the sheath 10 inside the body can be selected and adjusted by adjusting the adjusting component 41 outside the body, and a bidirectional bending can be realized. As an embodiment, the handle housing 50 is configured to facilitate gripping during operation. The handle housing 50 has an accommodating cavity 51, and the driving mechanism 30 is loaded in the accommodating cavity 51 of the handle housing 50. The handle housing 50 can simultaneously provide a protection function and a mounting support function for the components such as the driving mechanism 30. The handle housing 50 includes a bottom housing 52 and an upper housing 53 abutting the bottom housing 52 to form the accommodating cavity 51. Further, the outer surfaces of the upper housing 53 and the bottom housing 52 can both be provided with an anti-slip convex strip 55 so as to facilitate gripping by an operator.

As an embodiment, a plurality of reinforcing ribs 56 are provided on the inner wall of the handle housing 50, which may both enhance the strength of the handle housing 50 and provide a mounting support function for the components in the accommodating cavity 51 of the handle housing 50.

The proximal end of the sheath 10 runs through the handle housing 50 to facilitate connection with the driving mechanism 30 within the accommodating cavity 51 of the handle housing 50. The first traction wire 21 passes out of the inner wall of the sheath 10 in the accommodating cavity 51 of the handle housing 50 and is wound on the wire spool 322 of the first driven bevel gear 32 (referring in combination to Fig. 2), and the second traction wire 22 passes out of the inner wall of the sheath 10 in the accommodating cavity 51 of the handle housing 50 and is wound on the wire spool of the second driven bevel gear 33, so that both the connection of the first traction wire 21 with the first driven bevel gear 32 and the connection of the second traction wire 22 with the second driven bevel gear 33 can be realized. Meanwhile, the accommodating cavity 51 of the handle housing 50 may protect the first traction wire 21 and the second traction wire 22 therein. As a specific embodiment, the adjusting member 40 further includes an adjusting tube 42 having an end connected to the driving bevel gear 31. The sheath 10 may run through the adjusting tube 42. The adjusting component 41 is fixedly connected to the other end of the adjusting tube 42 via a key connection. The adjusting component 41 drives the adjusting tube 42 to rotate and may drive the driving bevel gear 31 to rotate synchronously so as to control the bending of the sheath 10.

At least two limiting members 421 are provided on the adjusting tube 42 to axially limit the adjusting tube 42. More specifically, the limiting members 421 are annular convex strips convexly arranged on the outer peripheral surface of the adjusting tube 42. Accordingly, limiting rib plates 501 and 502 bearing against the annular convex strips are provided on the inner wall of the handle housing 50 so as to limit and position both the forward movement and the backward movement of the adjusting tube 42 in the axial direction. As a specific embodiment, the two limiting members 421 may be located between the two limiting rib plates 501, 502. Of course, as an alternative embodiment, the two limiting rib plates 501, 502 may be located between the two limiting members 421 (not shown).

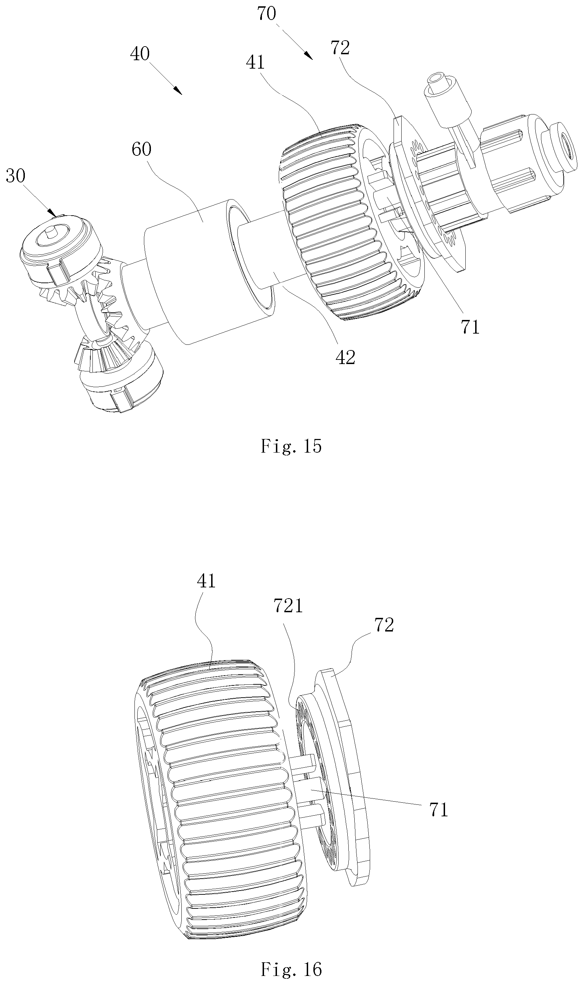

As an embodiment, the adjusting member 40 further includes a sleeve 60 rotatably surrounding the limiting members 421. The axial length of the limiting members 421 is less than the axial length of the sleeve 60, so as to reduce the contact area between the limiting members 421 and the sleeve 60, thereby reducing the friction between the contacting surfaces and enhancing the rotation performance. The sleeve 60 may, on the one hand, provide radial support to the adjusting tube 42 and enhance its radial load function to make up for the deficiency of large radial stress in bevel gear transmission; on the other hand, the sleeve 60 may reduce the friction resistance experienced when the adjusting tube 42 rotates as the sleeve 60 is rotatably connected to the adjusting tube 42, so that the adjusting tube 42 can rotate more smoothly while maintaining rigidity, thus saving labor and being lighter in adjustment. As an embodiment, referring to Figs. 15 and 16 in combination, the deflectable catheter 100 provided in the embodiment of the present application further includes a stop mechanism 70 arranged opposite to the adjusting component 41, and the stop mechanism 70 is configured to keep the bendable section 11 of the sheath 10 in a corresponding bent state after it is bent to a preset position, thus facilitating corresponding operations by a surgical operator.

In the embodiment of Figs. 15 to 17, the stop mechanism 70 includes a first stop 71 and a second stop 72, and one of the first stop 71 and the second stop 72 is connected to the driving bevel gear 31 and rotates synchronously with the driving bevel gear 31.

The other one of the first stop 71 and the second stop 72 remains stationary relative to the sheath 10. There is a stop force between the first stop 71 and the second stop 72, and the stop force enables the first stop 71 and the second stop 72 to limit circumferentially. The torque generated by the stop force on the driving bevel gear 31 is A1. When the sheath 10 is in a bent state, the torque generated by the pull-back force of the sheath 10 on the driving bevel gear 31 is A2, where A1 is greater than or equal to A2, so that the sheath 10 can be maintained in a certain bending state via the stop mechanism 70.

When the torque acting on the driving bevel gear 31 is greater than A1, the first stop 71 and the second stop 72 can rotate relative to each other to change the bending state of the sheath 10.

In the embodiment, the first stop 71 is connected to the driving bevel gear 31, and the first stop 71 can be circumferentially fixed to the driving bevel gear 31. The second stop 72 remains stationary relative to the sheath 10.

Specifically, in the embodiment, the first stop 71 is fixed to the adjusting component 41 such that the first stop 71 is connected to the driving bevel gear 31 via the adjusting component 41 and the adjusting tube 42. The first stop 71 can rotate synchronously with the adjusting tube 42 and/or the driving bevel gear 31.

In the embodiment, the second stop 72 can be fixed on the handle housing so as to keep the second stop 72 stationary relative to the sheath 10. The second stop 72 is spaced apart from and opposite to the adjusting component 41. The second stop 72 has a plurality of positioning recesses 721 arranged thereon in an annular layout. When the first stop 71 rotates synchronously with the adjusting component 41 and/or the driving bevel gear 31, it can be elastically snap-fitted with the positioning recess 721 at a corresponding position on the second stop 72 so that the adjusting component 41 is circumferentially limited. In addition, the adjusting component 41 and the adjusting tube 42 are circumferentially fixed to the driving bevel gear 31 (i.e., the adjusting component 41 and the adjusting tube 42 rotate synchronously with the driving bevel gear 31), so that the driving bevel gear 31 connected to the adjusting tube 42 is also circumferentially limited, thereby maintaining the sheath 10 in a corresponding bending state.

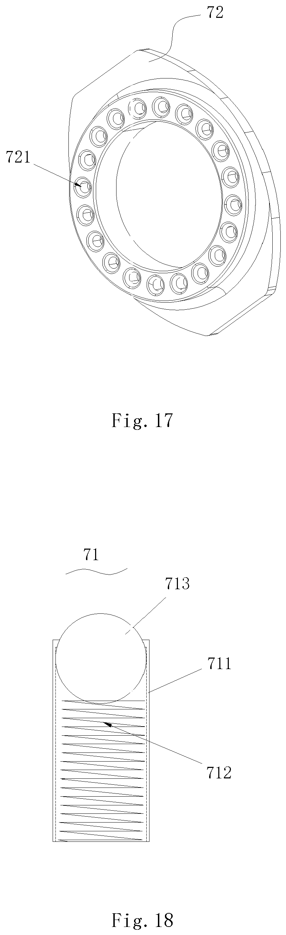

In one embodiment, referring to Fig. 18, the first stop 71 includes a stop tube 711 fixedly connected to the adjusting component 41 and an elastic member 712 and a ball 713 bearing against the elastic member 712 which are accommodated in the cavity of the stop tube 711, where a part of the ball 713 is exposed at the port of the stop tube 711 and faces the second stop 72. More specifically, the elastic member 712 may be a spring, and the elastic member 712 abuts against the ball 713 so as to force the ball 713 to move towards the second stop 72 to be engaged into a corresponding positioning recess 721 on the second stop 72, thereby realizing the stopping and positioning of the adjusting component 41 and the sheath 10. When the adjusting component 41 is rotated with a sufficiently large force, the torque acting on the driving bevel gear 31, which is larger than A1, may overcome the tension of the elastic member 712 so as to force the ball 713 to further compress the elastic member 712, and the ball 713 may retract back into the stop tube 711 and disengage from the positioning recess 721. Therefore, the circumferential limiting function of the first stop 71 with the second stop 72 is removed, and the adjusting component 41 may be adjusted to rotate continually for the next adjustment.

As a further embodiment, in the embodiment of the present application, the positions of the positioning recesses 721 on the first stop 71 and the second stop 72 can be interchanged, i.e., the first stop 71 can be fixedly connected to the second stop 72, and accordingly, the positioning recesses can be arranged on the adjusting component 41. This arrangement can also realize the function of positioning and maintaining the bending direction and bending angle of the sheath 10.

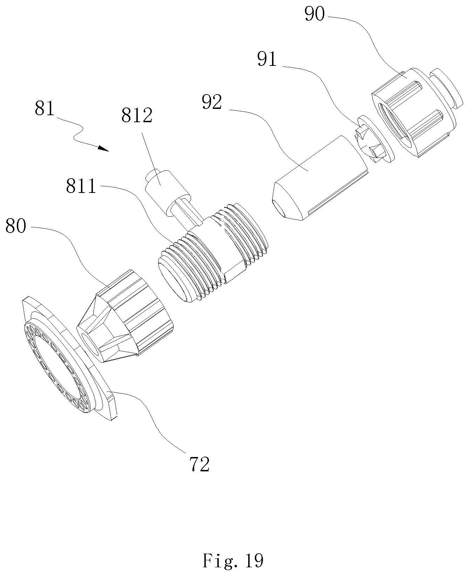

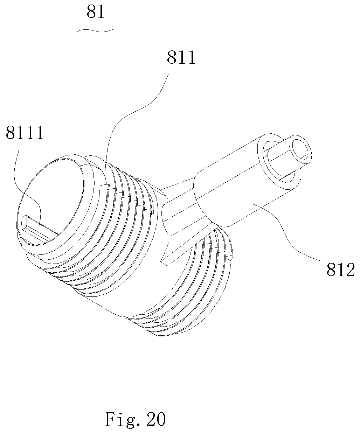

In the embodiment of Figs. 19 and 20, the deflectable catheter 100 provided in the embodiment of the present application further includes a connecting shaft sleeve 80 and a T-joint 81 extending through the middle of the second stop 72, where the T-joint 81 includes a transverse sleeve part 811 and an additional tube connecting part 812 arranged perpendicularly to the transverse sleeve part 811; one end of the transverse sleeve part 811 of the T-joint 81 is in threaded connection with the connecting shaft sleeve 80, and the other end is in threaded connection with a tail sleeve 90, and the additional tube connecting part 812 can be used for connecting with other accessories such as a hose. The tail sleeve 90 can not only close the proximal end of the handle housing 50, but also connect to other components as an intermediate adaptor, such as mating-connecting to a stent to be implanted or a delivery device.

As a specific embodiment, the proximal end port of the transverse sleeve part 811 of the T-joint 81 is provided with a seal ring 91 to seal the interior of the T-joint 81. The proximal end of the sheath 10 is snapped into the connecting shaft sleeve 80 and passes sequentially through the adjusting tube 42, the support frame 34, and then out of the distal end of the handle housing 50.

As an embodiment, a guide sleeve 92 is further provided in the transverse sleeve part 811 of the T-joint 81, where the guide sleeve 92 can be snapped inside the T-joint 81 via a key connection, the distal end of the guide sleeve 92 can be provided in the form of a tapered hole, and the hole size of the guide sleeve 92 can be correspondingly provided according to different hole sizes of a stent or a device or the like to be delivered, so as to provide a suitable delivery channel therefor.

### Second Embodiment:

As an alternative embodiment to the driving mechanism 30, the driving mechanism 30 may be configured to control the first traction wire 21 and the second traction wire 22 separately using two sets of bevel gear assemblies. Specifically, in the embodiment, the driving member includes a first driving bevel gear and a second driving bevel gear, where the first driving bevel gear and the second driving bevel gear are arranged coaxially but in reverse; the first driving bevel gear meshes with the first driven bevel gear, and the second driving bevel gear meshes with the second driven bevel gear. When the first driving bevel gear and the second driving bevel gear rotate, their rotation directions are the same, and according to the rotation transmission rule of the bevel gear pair, the first driven bevel gear 32 and the second driven bevel gear 33 driven by them have the same rotation direction as well. At this point, the winding direction of the first traction wire 21 on the first driven bevel gear 32 and the winding direction of the second traction wire 22 on the second driven bevel gear 33 are opposite. In this way, that when any of the first traction wire 21 and the second traction wire 22 is pulled, the other traction wire is correspondingly released, thereby controlling bidirectional bending of the sheath 10.

While the above embodiments are merely preferred embodiments of the present application, they are not intended to limit the present application. The invention is defined in the appended claims.

## Claims

1. A deflectable catheter (100), comprising:
a sheath (10) having a bendable section (11);
a first traction wire (21) anchored to the bendable section (11) of the sheath (10);
a second traction wire (22) anchored to the bendable section (11) of the sheath (10) at a location circumferentially different from that of the first traction wire (21);
a driving mechanism (30) connected to both the first traction wire (21) and the second traction wire (22), wherein the driving mechanism (30) pulls and releases the first traction wire (21) and the second traction wire (22), and when the driving mechanism (30) pulls any of the first traction wire (21) and the second traction wire (22), the driving mechanism (30) will synchronously release the other one of the first traction wire (21) and the second traction wire (22),
an adjusting member (40) and a handle housing (50);
wherein the adjusting member is configured to adjust the driving mechanism (30) for selecting and adjusting a bending direction and a bending angle of the sheath (10), the adjusting member (40) comprises an adjusting component (41) which is at least partially exposed outside the handle housing (50),
**characterized in that** the deflectable catheter (100) further comprises a stop mechanism (70) arranged opposite to the adjusting component (41) and is configured to maintain the bendable section (11) of the sheath (10) in a bent state.

2. The deflectable catheter (100) of claim 1, wherein the driving mechanism (30) comprises a driving member, a first driven member linked with the driving member, and a second driven member linked with the driving member, and wherein the first driven member is connected to the first traction wire (21), the second driven member (33) is connected to the second traction wire (22), and the driving member drives one of the first driven member and the second driven member to pull the traction wire (21, 22) connected thereto and synchronously drives the other one of the first driven member and the second driven member to release the traction wire connected thereto.

3. The deflectable catheter (100) of claim 2, wherein the driving member (31) is a driving bevel gear (31), the first driven member (32) is a first driven bevel gear (32) meshing with the driving bevel gear (31), the second driven member (33) is a second driven bevel gear (33) meshing with the driving bevel gear (31), the first driven bevel gear (32) is connected to the first traction wire (21), the second driven bevel gear (33) is connected to the second traction wire (22), the driving bevel gear (31) drives one of the first driven bevel gear (31) and the second driven bevel gear (32) to pull the traction wire (21,22) connected thereto and synchronously drives the other one of the first driven bevel gear (32) and the second driven bevel gear (33) to release the traction wire connected thereto.

4. The deflectable catheter (100) of claim 3, wherein the first traction wire (21) is wound on the first driven bevel gear (32) in a first direction, the second traction wire (22) is wound on the second driven bevel gear (33) in a second direction, and the first direction is in the same direction as the second direction; the driving bevel gear (31) drives the first driven bevel gear (32) and the second driven bevel gear (33) to rotate simultaneously, and rotation directions of the first driven bevel gear (32) and the second driven bevel gear (33) are opposite.

5. The deflectable catheter (100) of claim 3, wherein the first driven bevel gear (32) and the second driven bevel gear (33) are arranged symmetrically with respect to at least one axial plane of the driving bevel gear (31).

6. The deflectable catheter (100) of claim 5, wherein the first driven bevel gear (32) and the second driven bevel gear (33) are located at circumferentially different positions of the driving bevel gear (31), the rotational central axis of the first driven bevel gear (32) is arranged coaxially with that of the second driven bevel gear (33), and the rotational central axes of the first driven bevel gear (32) and the second driven bevel gear (33) are both perpendicular to the central axis of the driving bevel gear (31).

7. The deflectable catheter (100) of claim 3, wherein the driving mechanism (30) further comprises a support frame (34) comprising a first rotary shaft (342) and a second rotary shaft (343) which are arranged collinearly and located in the same radial plane of the sheath (10), the first driven bevel gear (32) is rotatably arranged on the first rotary shaft (342), and the second driven bevel gear (33) is rotatably arranged on the second rotary shaft (343); and particularly wherein rotatably contacting surfaces of the first rotary shaft (342) with the first driven bevel gear (32) are smooth surfaces, and rotatably contacting surfaces of the second rotary shaft (343) with the second driven bevel gear (33) are smooth surfaces.

8. The deflectable catheter (100) of any of claims 1-7, wherein the first traction wire (21) and the second traction wire (22) are located in the same axial plane of the sheath (10).

9. The deflectable catheter (100) of claim 3, the adjusting member (40) comprising an adjusting tube (42) having an end connected to the driving bevel gear (31), the adjusting component (41) is connected to the other end of the adjusting tube (42), wherein the sheath (10) extends through the adjusting tube (42), and the adjusting component (41) drives the adjusting tube (42) to rotate and drives the driving bevel gear (31) to rotate.

10. The deflectable catheter (100) of claim 9, the handle housing (50) having an accommodating cavity (51), wherein the driving mechanism (30) is located in the accommodating cavity (51) of the handle housing (50), at least two limiting members (421) are provided on the adjusting tube (42) to axially limit the adjusting tube (42), limiting rib plates (501,502) corresponding to and bearing against the two limiting members (421) are provided on an inner wall of the handle housing (50), the two limiting members (421) are located between the two limiting rib plates (501, 502), or the two limiting rib plates (501, 502) are located between the two limiting members (42).

11. The deflectable catheter (100) of claim 10, wherein the limiting members (421) protrude from an outer peripheral surface of the adjusting tube (42), the adjusting member (40) further comprises a sleeve (60) rotatably surrounding the limiting members (421), and an axial length of the limiting members (421) is less than an axial length of the sleeve (60).

12. The deflectable catheter (100) of claim 3, the stop mechanism (70) comprising a first stop (71) and a second stop (72), one of the first stop (71) and the second stop (72) is connected to the driving bevel gear (31) and rotates synchronously with the driving bevel gear (31), the other one of the first stop (71) and the second stop (72) remains stationary relative to the sheath (70), there is a stop force between the first stop (71) and the second stop (72), and the stop force enables the first stop (71) and the second stop (72) to limit each other circumferentially, a torque generated by the stop force on the driving bevel gear (31) is A1, and when the sheath (10) is in a bent state, a torque generated by a pull-back force of the sheath (10) on the driving bevel gear (31) is A2, wherein A1 is greater than or equal to A2.

13. The deflectable catheter (100) of claim 12, wherein the first stop (71) and the second stop (72) rotate relative to each other when a torque acting on the driving bevel gear (31) is greater than A1.

14. The deflectable catheter (100) of claim 13, wherein the first stop (71) is connected to the driving bevel gear (31), the first stop (71) rotates synchronously with the driving bevel gear (31), the second stop (72) has a plurality of positioning recesses (721) arranged thereon in an annular layout, and the first stop (71) rotates synchronously with the driving bevel gear (31) and is elastically snap-fitted with a corresponding one of the plurality of positioning recesses (721) on the second stop (72); and particularly wherein the first stop (71) comprises a stop tube (711) fixedly connected to the driving bevel gear (31), and an elastic member (712) and a ball (713) bearing against the elastic member (712) which are accommodated in a cavity of the stop tube (711), and when a part of the ball (713) is exposed at a port of the stop tube (711) and faces the positioning recess (721), the elastic member (712) will elastically press the ball (713) to push it into the positioning recess (721).

15. The deflectable catheter (100) of claim 2, wherein the driving member comprises a first driving bevel gear and a second driving bevel gear, the first driven member is a first driven bevel gear (32) meshing with the first driving bevel gear, the second driven member is a second driven bevel gear (33) meshing with the second driving bevel gear, the first traction wire (21) is wound on the first driven bevel gear (32) in a first direction, the second traction wire (22) is wound on the second driven bevel gear (33) in a second direction, the first direction is opposite to the second direction, and rotation directions of the first driven bevel gear (32) and the second driven bevel gear (33) are the same.

## Patentansprüche

1. Ein biegsamer Katheder (100), der folgendes aufweist:
eine Hülle (10) mit einem biegbaren Abschnitt (11);
einen ersten Traktionsdraht (21), der an dem biegsamen Abschnitt (11) der Hülle (10) verankert ist;
einen zweiten Traktionsdraht (22), der an dem biegsamen Abschnitt (11) der Hülle (10) an einer Position verankert ist, die sich in Umfangsrichtung von derjenigen des ersten Traktionsdrahtes (21) unterscheidet;
einen Treibmechanismus (30), der sowohl mit dem ersten Traktionsdraht (21) als auch mit dem zweiten Traktionsdraht (22) verbunden ist, wobei der Treibmechanismus (30) den ersten Traktionsdraht (21) und den zweiten Traktionsdraht (22) zieht und freigibt, und wenn der Treibmechanismus (30) einen von dem ersten Traktionsdraht (21) und dem zweiten Traktionsdraht (22) zieht, der Treibmechanismus (30) synchron den anderen von dem ersten Traktionsdraht (21) und dem zweiten Traktionsdraht (22) freigibt,
ein Anpassungselement (40) und ein Griffgehäuse (50);
wobei das Anpassungselement konfiguriert ist, um den Treibmechanismus (30) zum Auswählen und Einstellen einer Biege-Richtung und eines Biege-Winkels der Hülle (10) einzustellen, wobei das Anpassungselement (40) eine Anpassungskomponente (41) aufweist, die zumindest teilweise außerhalb des Griffgehäuses (50) freigelegt ist,
**dadurch gekennzeichnet, dass** der biegsame Katheder (100) ferner einen Anschlagmechanismus (70) aufweist, der gegenüber der Anpassungskomponente (41) angeordnet und konfiguriert ist, um den biegsamen Abschnitt (11) der Hülle (10) in einem gebogenen Zustand zu halten.

2. Der biegsame Katheder (100) nach Anspruch 1, wobei der Treibmechanismus (30) ein treibendes Element, ein erstes getriebenes Element, das mit dem treibenden Element verbunden ist, und ein zweites getriebenes Element aufweist, das mit dem treibenden Element verbunden ist, und wobei das erste getriebene Element mit dem ersten Traktionsdraht (21) verbunden ist, das zweite getriebene Element (33) mit dem zweiten Traktionsdraht (22) verbunden ist, und das treibende Element eines von dem ersten getriebenen Element und dem zweiten getriebenen Element antreibt, um den damit verbundenen Traktionsdraht (21, 22) zu ziehen, und synchron das andere von dem ersten getriebenen Element und dem zweiten getriebenen Element antreibt, um den damit verbundenen Traktionsdraht freizugeben.

3. Der biegsame Katheder (100) nach Anspruch 2, wobei das Antriebselement (31) ein treibendes Kegelrad (31) ist, das erste getriebene Element (32) ein erstes getriebenes Kegelrad (32) ist, das mit dem treibenden Kegelrad (31) kämmt, das zweite getriebene Element (33) ein zweites getriebenes Kegelrad (33) ist, das mit dem treibenden Kegelrad (31) kämmt, das erste getriebene Kegelrad (32) mit dem ersten Traktionsdraht (21) verbunden ist, das zweite getriebene Kegelrad (33) mit dem zweiten Traktionsdraht (22) verbunden ist, das treibende Kegelrad (31) eines von dem ersten getriebenen Kegelrad (31) und dem zweiten getriebenen Kegelrad (32) betreibt, um den damit verbundenen Traktionsdraht (21, 22) zu ziehen, und synchron das andere von dem ersten getriebenen Kegelrad (32) und dem zweiten getriebenen Kegelrad (33) antreibt, um den damit verbundenen Traktionsdraht zu lösen.

4. Der biegsame Katheder (100) nach Anspruch 3, wobei der erste Traktionsdraht (21) auf das erste getriebene Kegelrad (32) in einer ersten Richtung gewickelt ist, der zweite Traktionsdraht (22) auf das zweite getriebene Kegelrad (33) in einer zweiten Richtung gewickelt ist, und die erste Richtung in der gleichen Richtung wie die zweite Richtung liegt; das treibende Kegelrad (31) das erste getriebene Kegelrad (32) und das zweite getriebene Kegelrad (33) gleichzeitig in Drehung versetzt und die Drehrichtungen des ersten getriebenen Kegelrads (32) und des zweiten getriebenen Kegelrads (33) entgegengesetzt sind.

5. Der biegsame Katheder (100) nach Anspruch 3, wobei das erste getriebene Kegelrad (32) und das zweite getriebene Kegelrad (33) symmetrisch zu mindestens einer Axialebene des treibenden Kegelrads (31) angeordnet sind.

6. Der biegsame Katheder (100) nach Anspruch 5, wobei das erste getriebene Kegelrad (32) und das zweite getriebene Kegelrad (33) an in Umfangsrichtung unterschiedlichen Positionen des treibenden Kegelrades (31) angeordnet sind, die Rotationsmittelachse des ersten getriebenen Kegelrads (32) derart ausgebildet ist, dass sie mit der des zweiten getriebenen Kegelrads (33) koaxial ist, und die Rotationsmittelachsen des ersten getriebenen Kegelrads (32) und des zweiten getriebenen Kegelrads (33) beide senkrecht zur Mittelachse des treibenden Kegelrads (31) stehen.

7. Der biegsame Katheder (100) nach Anspruch 3, wobei der Treibmechanismus (30) ferner einen Stützrahmen (34) aufweist, der eine erste rotierende Welle (342) und eine zweite rotierende Welle (343) aufweist, die kollinear und in der gleichen radialen Ebene der Hülle (10) ausgebildet sind, wobei das erste getriebene Kegelrad (32) drehbar auf der ersten rotierenden Welle (342) angeordnet ist und das zweite getriebene Kegelrad (33) drehbar auf der zweiten rotierenden Welle (343) angeordnet ist; und wobei insbesondere drehbare Kontaktflächen der ersten rotierenden Welle (342) mit dem ersten getriebenen Kegelrad (32) glatte Flächen sind, und drehbare Kontaktflächen der zweiten rotierenden Welle (343) mit dem zweiten getriebenen Kegelrad (33) glatte Flächen sind.

8. Der biegsame Katheder (100) nach einem der Ansprüche 1 bis 7, wobei der erste Traktionsdraht (21) und der zweite Traktionsdraht (22) in der gleichen axialen Ebene der Hülle (10) positioniert sind.

9. Der biegsame Katheder 100) nach Anspruch 3, wobei das Anpassungselement (40) ein Anpassungsrohr (42) aufweist, dessen einer Endabschnitt mit dem treibenden Kegelrad (31) verbunden ist, wobei die Anpassungskomponente (41) mit dem anderen Endabschnitt des Anpassungsrohr (42) verbunden ist, wobei sich die Hülle (10) durch das Anpassungsrohr (42) erstreckt und die Anpassungskomponente (41) das Anpassungsrohr (42) in Drehung versetzt und das treibende Kegelrad (31) in Drehung versetzt.

10. Der biegsame Katheder (100) nach Anspruch 9, wobei das Griffgehäuse (50) einen aufnehmenden Hohlraum (51) aufweist, wobei der Anpassungsmechanismus (30) in dem aufnehmenden Hohlraum (51) des Griffgehäuses (50) positioniert ist, wobei mindestens zwei begrenzende Elemente (421) an dem Anpassungsrohr (42) vorgesehen sind, um den Anpassungsrohr (42) axial zu begrenzen, an einer inneren Wand des Griffgehäuses (50) Begrenzungsrippenplatten (501, 502) vorgesehen sind, die den beiden begrenzenden Elementen (421) entsprechen und sich an diesen abstützen, die beiden begrenzenden Elemente (421) zwischen den beiden Begrenzungsrippenplatten (501, 502) angeordnet sind, oder die beiden Begrenzungsrippenplatten (501, 502) zwischen den beiden begrenzenden Elementen (42) angeordnet sind.

11. Der biegsame Katheder (100) nach Anspruch 10, wobei die begrenzenden Elemente (421) von einer äußeren Umfangsfläche des Anpassungsrohrs (42) vorstehen, das Anpassungselement (40) ferner eine Hülse (60) aufweist, die die begrenzenden Elemente (421) drehbar umgibt, und eine axiale Länge der begrenzenden Elemente (421) geringer ist als eine axiale Länge der Hülse (60).

12. Der biegsame Katheder (100) nach Anspruch 3, wobei der Anschlagmechanismus (70) einen ersten Anschlag (71) und einen zweiten Anschlag (72) aufweist, wobei einer von dem ersten Anschlag (71) und dem zweiten Anschlag (72) mit dem treibenden Kegelrad (31) verbunden ist und sich synchron mit dem treibenden Kegelrad (31) dreht, wobei der andere von dem ersten Anschlag (71) und dem zweiten Anschlag (72) relativ zu der Hülle (70) stationär bleibt, eine Anschlagskraft zwischen dem ersten Anschlag (71) und dem zweiten Anschlag (72) vorhanden ist und die Anschlagskraft es dem ersten Anschlag (71) und dem zweiten Anschlag (72) ermöglicht, sich gegenseitig in Umfangsrichtung zu begrenzen, ein durch die Anschlagskraft auf das treibende Kegelrad (31) erzeugtes Drehmoment A1 ist und, wenn sich die Hülle (10) in einem gebogenen Zustand befindet, ein durch eine Rückzugskraft der Hülle (10) auf das treibende Kegelrad (31) erzeugtes Drehmoment A2 ist, wobei A1 größer oder gleich A2 ist.

13. Der biegsame Katheder (100) nach Anspruch 12, wobei der erste Anschlag (71) und der zweite Anschlag (72) sich relativ zueinander drehen, wenn ein auf das treibende Kegelrad (31) wirkendes Drehmoment größer als A1 ist.

14. Der biegsame Katheder (100) nach Anspruch 13, wobei der erste Anschlag (71) mit dem treibenden Kegelrad (31) verbunden ist, der erste Anschlag (71) synchron mit dem treibenden Kegelrad (31) rotiert, der zweite Anschlag (72) eine derart ausgebildete Vielzahl von Positionierausnehmungen (721) aufweist, und der erste Anschlag (71) synchron mit dem treibenden Kegelrad (31) rotiert und mit einer entsprechenden der Vielzahl von Positionierausnehmungen (721) am zweiten Anschlag (72) elastisch verrastet ist; und wobei der erste Anschlag (71) insbesondere ein Anschlagrohr (711) aufweist, das fest mit dem treibenden Kegelrad (31) verbunden ist, und ein elastisches Element (712) und eine Kugel (713), die sich an dem elastischen Element (712) abstützt, die in einem Hohlraum des Anschlagrohrs (711) untergebracht sind, und wenn ein Teil der Kugel (713) an einem Anschluss des Anschlagrohrs (711) freiliegt und der Positionierausnehmung (721) zugewandt ist, wird das elastische Element (712) die Kugel (713) elastisch drücken, um sie in die Positionierausnehmung (721) zu schieben.

15. Der biegsame Katheder (100) nach Anspruch 2, wobei das Treibelement ein erstes treibendes Kegelrad und ein zweites treibendes Kegelrad aufweist, das erste getriebene Element ein erstes getriebenes Kegelrad (32) ist, das mit dem ersten treibenden Kegelrad kämmt, das zweite getriebene Element ein zweites getriebenes Kegelrad (33) ist, das mit dem zweiten treibenden Kegelrad kämmt, der erste Traktionsdraht (21) auf das erste getriebene Kegelrad (32) in einer ersten Richtung gewickelt ist, der zweite Traktionsdraht (22) auf das zweite getriebene Kegelrad (33) in einer zweiten Richtung gewickelt ist, die erste Richtung der zweiten Richtung entgegengesetzt ist, und die Drehrichtungen des ersten getriebenen Kegelrads (32) und des zweiten getriebenen Kegelrads (33) gleich sind.

## Revendications

1. Cathéter déformable (100) comprenant :
une gaine (10) présentant une section pouvant être courbée (11) ;
un premier fil de traction (21) ancré à la section pouvant être courbée (11) de la gaine (10) ;
un second fil de traction (22) ancré à la section pouvant être courbée (11) de la gaine (10) au niveau d'un emplacement circonférentiellement différent de celui du premier fil de traction (21) ;
un mécanisme d'entraînement (30) relié à la fois au premier fil de traction (21) et au second fil de traction (22), ledit mécanisme d'entraînement (30) tirant et libérant le premier fil de traction (21) et le second fil de traction (22), et lorsque le mécanisme d'entraînement (30) tire l'un quelconque fil parmi le premier fil de traction (21) et le second fil de traction (22), ledit mécanisme d'entraînement (30) libérant de manière synchrone l'autre fil parmi le premier fil de traction (21) et le second fil de traction (22),
un élément de réglage (40) et un boîtier de poignée (50) ;
ledit élément de réglage étant conçu pour régler le mécanisme d'entraînement (30) de manière à sélectionner et régler une direction de courbure et un angle de courbure de la gaine (10), ledit élément de réglage (40) comprenant un composant de réglage (41) qui est au moins partiellement exposé à l'extérieur du boîtier de poignée (50),
**caractérisé en ce que** le cathéter déformable (100) comprend en outre un mécanisme à butées (70) disposé face au composant de réglage (41) et est conçu pour maintenir la section pouvant être courbée (11) de la gaine (10) dans un état courbé.

2. Cathéter déformable (100) selon la revendication 1, ledit mécanisme d'entraînement (30) comprenant un élément d'entraînement, un premier élément entraîné relié à l'élément d'entraînement et un second élément entraîné relié à l'élément d'entraînement, et ledit premier élément entraîné étant relié au premier fil de traction (21), ledit second élément entraîné (33) étant relié au second fil de traction (22), et ledit élément d'entraînement entraînant un élément parmi le premier élément entraîné et le second élément entraîné de manière à tirer le fil de traction (21, 22) relié à celui-ci et entraînant de manière synchrone l'autre élément parmi le premier élément entraîné et le second élément entraîné de manière à libérer le fil de traction relié à celui-ci.

3. Cathéter déformable (100) selon la revendication 2, ledit élément d'entraînement (31) étant un engrenage conique d'entraînement (31), ledit premier élément entraîné (32) étant un premier engrenage conique entraîné (32) s'engrenant avec l'engrenage conique d'entraînement (31), ledit second élément entraîné (33) étant un second engrenage conique entraîné (33) s'engrenant avec l'engrenage conique d'entraînement (31), ledit premier engrenage conique entraîné (32) étant relié au premier fil de traction (21), ledit second engrenage conique entraîné (33) étant relié au second fil de traction (22), ledit engrenage conique d'entraînement (31) entraînant un engrenage parmi le premier engrenage conique entraîné (31) et le second engrenage conique entraîné (32) de manière à tirer le fil de traction (21, 22) qui est relié à celui-ci et entraînant de manière synchrone l'autre engrenage parmi le premier engrenage conique entraîné (32) et le second engrenage conique entraîné (33) de manière à libérer le fil de traction qui est relié à celui-ci.

4. Cathéter déformable (100) selon la revendication 3, ledit premier fil de traction (21) étant enroulé sur le premier engrenage conique entraîné (32) dans un premier sens, ledit second fil de traction (22) étant enroulé sur le second engrenage conique entraîné (33) dans un second sens, et ledit premier sens étant dans le même sens que le second sens ; ledit engrenage conique d'entraînement (31) entraînant le premier engrenage conique entraîné (32) et le second engrenage conique entraîné (33) de manière à ce qu'ils tournent simultanément, et lesdits sens de rotation du premier engrenage conique entraîné (32) et du second engrenage conique entraîné (33) étant opposées.

5. Cathéter déformable (100) selon la revendication 3, ledit premier engrenage conique entraîné (32) et ledit second engrenage conique entraîné (33) étant disposés symétriquement par rapport à au moins un plan axial de l'engrenage conique d'entraînement (31).

6. Cathéter déformable (100) selon la revendication 5, ledit premier engrenage conique entraîné (32) et ledit second engrenage conique entraîné (33) étant situés au niveau de positions circonférentiellement différentes de l'engrenage conique d'entraînement (31), ledit axe central de rotation du premier engrenage conique entraîné (32) étant disposé de manière coaxiale par rapport à celui du second engrenage conique entraîné (33), et lesdits axes centraux de rotation du premier engrenage conique entraîné (32) et du second engrenage conique entraîné (33) étant tous deux perpendiculaires à l'axe central de l'engrenage conique d'entraînement (31).

7. Cathéter déformable (100) selon la revendication 3, ledit mécanisme d'entraînement (30) comprenant en outre un cadre support (34) comprenant un premier arbre rotatif (342) et un second arbre rotatif (343) qui sont disposés de manière colinéaire et situés dans le même plan radial de la gaine (10), ledit premier engrenage conique entraîné (32) étant disposé de manière rotative sur le premier arbre rotatif (342), et ledit second engrenage conique entraîné (33) étant disposé de manière rotative sur le second arbre rotatif (343) ; et en particulier lesdites surfaces en contact de manière rotative du premier arbre rotatif (342) avec le premier engrenage conique entraîné (32) étant des surfaces lisses, et lesdites surfaces en contact de manière rotative du second arbre rotatif (343) avec le second engrenage conique entraîné (33) étant des surfaces lisses.

8. Cathéter déformable (100) selon l'une quelconque des revendications 1 à 7, ledit premier fil de traction (21) et ledit second fil de traction (22) étant situés dans le même plan axial de la gaine (10).

9. Cathéter déformable (100) selon la revendication 3, ledit élément de réglage (40) comprenant un tube de réglage (42) possédant une extrémité reliée à l'engrenage conique d'entraînement (31), ledit composant de réglage (41) étant relié à l'autre extrémité du tube de réglage (42), ladite gaine (10) s'étendant à travers le tube de réglage (42), et ledit composant de réglage (41) entraînant le tube de réglage (42) en rotation et entraînant l'engrenage conique d'entraînement (31) en rotation.

10. Cathéter déformable (100) selon la revendication 9, ledit boîtier de poignée (50) comportant une cavité de réception (51), ledit mécanisme d'entraînement (30) étant situé dans la cavité de réception (51) du boîtier de poignée (50), au moins deux éléments de limitation (421) étant pourvus sur le tube de réglage (42) de manière à limiter axialement le tube de réglage (42), des plaques à nervure de limitation (501, 502) correspondant aux deux éléments de limitation (421) et s'appuyant contre ceux-ci étant pourvues sur une paroi interne du boîtier de poignée (50), lesdits deux éléments de limitation (421) étant situés entre les deux plaques à nervure de limitation (501, 502), ou lesdites deux plaques à nervure de limitation (501, 502) étant situées entre les deux éléments de limitation (42).

11. Cathéter déformable (100) selon la revendication 10, lesdits éléments de limitation (421) faisant saillie depuis une surface périphérique externe du tube de réglage (42), ledit élément de réglage (40) comprenant en outre un manchon (60) entourant de manière rotative les éléments de limitation (421), et une longueur axiale des éléments de limitation (421) étant inférieure à une longueur axiale du manchon (60).

12. Cathéter déformable (100) selon la revendication 3, ledit mécanisme à butées (70) comprenant une première butée (71) et une seconde butée (72), une butée parmi la première butée (71) et la seconde butée (72) étant reliée à l'engrenage conique d'entraînement (31) et tournant de manière synchrone avec l'engrenage conique d'entraînement (31), l'autre butée parmi la première butée (71) et la seconde butée (72) restant immobile par rapport à la gaine (70), une force de butée existant entre la première butée (71) et la seconde butée (72), et ladite force de butée permettant à la première butée (71) et à la seconde butée (72) de se limiter l'une l'autre de manière circonférentielle, un couple généré par la force de butée sur l'engrenage conique d'entraînement (31) étant A1, et lorsque la gaine (10) se trouve dans un état courbé, un couple généré par une force de rappel de la gaine (10) sur l'engrenage conique d'entraînement (31) étant A2, A1 étant supérieur ou égal à A2.

13. Cathéter déformable (100) selon la revendication 12, ladite première butée (71) et ladite seconde butée (72) tournant l'une par rapport à l'autre lorsqu'un couple agissant sur l'engrenage conique d'entraînement (31) est supérieur à A1.

14. Cathéter déformable (100) selon la revendication 13, ladite première butée (71) étant reliée à l'engrenage conique d'entraînement (31), ladite première butée (71) tournant de manière synchrone avec l'engrenage conique d'entraînement (31), ladite seconde butée (72) comportant une pluralité d'évidements de positionnement (721) disposés sur celle-ci selon une disposition annulaire, et ladite première butée (71) tournant de manière synchrone avec l'engrenage conique d'entraînement (31) et étant élastiquement encliquetée avec un évidement correspondant parmi la pluralité d'évidements de positionnement (721) sur la seconde butée (72) ; et en particulier ladite première butée (71) comprenant un tube de butée (711) relié de manière fixe à l'engrenage conique d'entraînement (31), et un élément élastique (712) et une bille (713) s'appuyant contre l'élément élastique (712) qui sont logés dans une cavité du tube de butée (711), et lorsqu'une partie de la bille (713) est exposée au niveau d'un orifice du tube de butée (711) et fait face à l'évidement de positionnement (721), ledit élément élastique (712) comprimant élastiquement la bille (713) de manière à la pousser dans l'évidement de positionnement (721).

15. Cathéter déformable (100) selon la revendication 2, ledit élément d'entraînement comprenant un premier engrenage conique d'entraînement et un second engrenage conique d'entraînement, ledit premier élément entraîné étant un premier engrenage conique entraîné (32) s'engrenant avec le premier engrenage conique d'entraînement, ledit second élément entraîné étant un second engrenage conique entraîné (33) s'engrenant avec le second engrenage conique d'entraînement, ledit premier fil de traction (21) étant enroulé sur le premier engrenage conique entraîné (32) dans un premier sens, ledit second fil de traction (22) étant enroulé sur le second engrenage conique entraîné (33) dans un second sens, ledit premier sens étant opposé au second sens, et lesdits sens de rotation du premier engrenage conique entraîné (32) et du second engrenage conique entraîné (33) étant les mêmes.
